# EUROPEAN PATENT SPECIFICATION

(11) **EP 3 236 952 B1**
(45) Date of publication and mention of the grant of the patent: **03.07.2019**
(21) Application number: 15820858.7
(22) Date of filing: 23.12.2015
(51) Int. Cl.: A61K 9/24, A61K 9/20, A61K 9/28

(54) **PHARMACEUTICAL TABLET COMPOSITION**
PHARMAZEUTISCHE TABLETTENZUBEREITUNG
COMPOSITION DRAGEE PHARMACEUTIQUE

(30) Priority: 23.12.2014 SI 201400454
(43) Date of publication of application: 01.11.2017
(73) Proprietor: KRKA, d.d., Novo mesto, 8501 Novo mesto (SI)
(72) Inventor: KUKEC, Simon, 1290 Grosupljem (SI); VRECER, Franc, 8351 Straza (SI); STANGELJ, Veronika, 8322 Stopice (SI); HUDOVORNIK, Grega, 8000 Novo mesto (SI); SKUBE, Maja Kincl, 8000 Novo mesto (SI); SLANC-VOVK, Janika, 1000 Ljublijana (SI); SIMONCIC, Kristina Debeljak, 8310 Sentjernej (SI); PAVLIN, Darja, 8351 Straza (SI)
(74) Representative: Hoefer & Partner Patentanwälte mbB
(86) International application number: PCT/EP2015/081147
(87) International publication number: WO 2016/102661

(56) References cited:
- WO-A1-2009/118764
- WO-A1-2012/144964
- US-A1- 2004 022 846
- US-A1- 2008 166 407
- US-A1- 2008 214 619
- US-A1- 2012 064 159
- US-A1- 2013 243 859
- US-B1- 6 544 556

## Description

### FIELD OF THE INVENTION

The present invention relates to a pharmaceutical tablet composition comprising a non-steroidal anti-inflammatory drug and a proton pump inhibitor.

### BACKGROUND OF THE INVENTION

It is known that combined use of a non-steroidal anti-inflammatory drug (NSAID) and a proton pump inhibitor (PPI) has reduced risk of ulcerations of upper part of gastrointestinal tract due to NSAID use compared with NSAIDs taken alone. There are many examples in prior art of fixed dose pharmaceutical compositions comprising an NSAID and a PPI.

VIMOVO modified-release tablets comprise delayed release (DR) naproxen and immediate release (IR) esomeprazole. VIMOVO has been shown to significantly decrease the occurrence of gastric ulcers and NSAID associated upper gastrointestinal adverse events compared to naproxen alone. Due to sensitivity of a PPI to an acid environment such formulations are not optimal because large portion of the PPI is degraded by gastric acid leading to a reduced bioavailability of the PPI and presence of increased amounts of PPI's degradation products which could result in harmful adverse effects on the user. WO02098352, Indian patent application 2679/CHE/2009, US2010305163 and Korean patent application KR1020120038030 teach the preparation of formulations comprising a NSAID and an immediate release PPI and having above mentioned drawbacks.

WO9725064 discloses oral pharmaceutical dosage forms comprising a PPI and one or more NSAIDs in a fixed formulation, wherein the PPI is protected by an enteric coating layer. The fixed formulation is in the form of an enteric coating layered tablet, a capsule or a multiple unit tableted dosage form. The problem with multiple unit tableted dosage forms as disclosed in WO9725064 is in potential cracking of the enteric coating layer during tableting. Majority of formulations as disclosed in WO9725064 comprise the NSAID in an immediate release form which is potentially disadvantageous due to direct contact between the NSAID and gastric mucosa leading to erosion and bleeding of gastric mucosa and consequently stomach upset and discomfort. The enteric coating layered tablet of example 6 can have potential stability problems due to interaction between omeprazole magnesium and diclofenac sodium which are together mixed and granulated and later compressed into a tablet core. Furthermore, mixture of NSAID and PPI of example 6 cannot achieve the level of independent release rate of NSAID and PPI as in the multilayer tablet of the present invention.

Article IJPRD 2011, vol. 3(8), 160-169 discloses sustained release mucoadhesive tablets comprising mixture of naproxen sodium and pantoprazole sodium. These tablets have the same problem as described hereinabove, namely direct contact between the NSAID and the PPI.

WO0222108 discloses solid oral dosage forms comprising diclofenac extended release tablet and an enteric coated PPI without a separating layer between the PPI and the enteric coating. Absence of the separating layer between the PPI and enteric coating is potentially detrimental to the stability of PPI because it enables the interaction between the acidic polymer and the acid sensitive drug.

US 2004/022846 A1 describes an oral pharmaceutical dosage form comprising an acid susceptible proton pump inhibitor and one or more NSAIDs in a fixed formulation, wherein the proton pump inhibitor is protected by an enteric coating layer. The fixed formulation is in the form of an enteric coating layered tablet, a capsule or a multiple unit tableted dosage form.

US 2008/214619 A1 discloses pharmaceutical proton pump inhibitor (PPI) medications and methods for preventing and/or treating gastrointestinal disorders characterized by abnormalities in gastric acid secretion at anytime of the day or night without the need for food effect or to be taken with food.

US20080166407 discloses tablets comprising an NSAID, a PPI, a thermoplastic hydrophilic polymer, a microparticulate excipient and a cellulosic enteric coating. There is no separating layer between the PPI and enteric coating which is potentially problematic due to stability issues.
The present invention provides pharmaceutical compositions overcoming the deficiencies and disadvantages of the prior art pharmaceutical compositions described hereinabove.

### BRIEF DESCRIPTION OF THE DRAWINGS

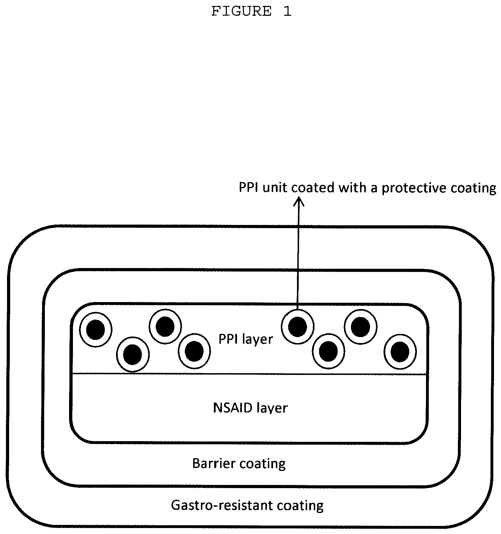

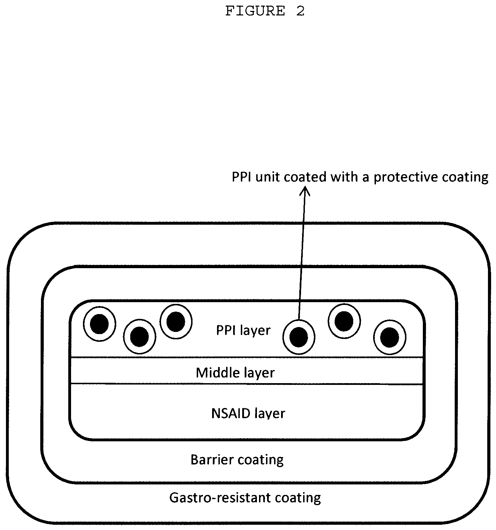

Figure 1 and 2 illustrate tablet dosage forms according to the present invention.

### DESCRIPTION OF THE INVENTION

The object of the present invention is a pharmaceutical tablet composition comprising an NSAID and a PPI and having improved stability and bioavailability. The present invention relates to a pharmaceutical tablet composition comprising:
a) a multilayer tablet core comprising:
   i) a non-steroidal anti-inflammatory drug (NSAID) layer comprising an NSAID and at least one pharmaceutically acceptable excipient;
   ii) an optional middle layer;
   iii) a proton pump inhibitor (PPI) layer comprising multiple units of PPI coated with a protective coating;
b) a barrier coating surrounding the multilayer tablet core; and
c) a gastro-resistant coating surrounding the barrier coating.

The term "multilayer tablet", as used herein, refers to a pharmaceutical tablet which is made up of at least two distinct layers, such as at least two layers, at least three layers, at least four layers, at least five layers, etc., with the individual layers being arranged one on top of another. The multilayer tablet generally may have a sandwich-like appearance because the edges of each layer may be exposed (not taking into account any surrounding coatings which may be present). Typically, adjacent layers of the tablet will be of different composition. The term "multilayer tablet core", as used herein, can in particular refer to a tablet core which is made up of at least two distinct layers, such as at least two layers, at least three layers, at least four layers, at least five layers, etc., with the individual layers being arranged one on top of another. The multilayer tablet core generally has a sandwich-like appearance because the edges of each layer are exposed. Typically, adjacent layers of the tablet core will be of different composition. The term "pharmaceutical tablet composition" encompasses in particular pharmaceutical tablets.

The term "NSAID", as used herein, refers to any compound acting as a non-steroidal anti-inflammatory agent identifiable as such by one of ordinary skill in the art. The NSAID can be in a free acid or a salt form. By the term "free acid" it is meant that the NSAID includes an acidic group which is not derivatised for example by the formation of a salt. The term "NSAID" includes, but is not limited to, propionic acid derivatives such as naproxen, ibuprofen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, bucloxic acid; acetic acid derivatives such as indomethacin, sulindac, tolmetin, zomepirac, diclofenac, fenchlofenac, alchlofenac, ibufenac, isoxepac, furofenac, tiopinac, zidometacin, acemetacin, fentiazac, clidanac, oxipinac; enolic acid derivatives such as piroxicam, meloxicam, tenoxicam, droxicam, lomoxicam, isoxicam; fenamic acid derivatives such as mefenamic acid, meclofenamic acid, flufenamic acid, tolfenamic acid; COX-2 inhibitors such as celecoxib, etoricoxib, lumiracoxib, parecoxib, rofecoxib, rofecoxib, valdecoxib; salicylic acid, acetylsalicylic acid, methyl salicylate, acetaminophen and/or salts and enantiomers thereof. Preferably, the NSAID is selected from the group consisting of naproxen, ibuprofen, diclofenac, ketoprofen, acetylsalicylic acid and/or salts and enantiomers thereof. Suitable salts are for example naproxen sodium, ibuprofen sodium, diclofenac sodium and/or ibuprofen lysinate. More preferably, the NSAID is selected from the group consisting of naproxen, ibuprofen, diclofenac and/or enantiomers thereof. Even more preferably, the NSAID is naproxen and/or naproxen sodium, most preferred NSAID is naproxen. It is preferred that 95 weight % of particles of NSAID used in formulation of tablet composition according to the present invention is below 400*µ*m, preferably below 300 *µ*m and most preferably below 250 *µ*m. In case naproxen is used as an NSAID, its particle size can be limited to a size wherein at least 80 weight % of particles are smaller than 100 *µ*m, preferably smaller than 80 *µ*m. The particle size distribution was determined by means of sieve analysis. The amount of NSAID in a NSAID layer is preferably at least 70% w/w, more preferably at least 80% w/w and most preferably at least 90% w/w.

The term "NSAID layer", as used herein, refers to a layer in a multilayer tablet which comprises an NSAID. The NSAID layer, according to the present invention, comprises at least one pharmaceutically acceptable excipient which can be selected from the group consisting of diluents, binders, disintegrants, glidants, lubricants and/or pigments. In a special embodiment the NSAID layer can comprise one or more controlled release excipients homogenously admixed with NSAID drug particles in a way to effectively prolong the release of said NSAID to at least six, preferably 8 and most preferably to at least 12 hours. The dissolution profiles of the individual tablets can be determined according to method described in European Pharmacopoeia 7.0, chapter 2.9.3.

Preferably, the NSAID layer comprises a or at least one pharmaceutically acceptable excipient selected from the group consisting of croscarmellose sodium, crospovidone, silica, magnesium stearate, and/or povidone. Most preferably, the excipient is croscarmellose sodium. The NSAID layer can comprise croscarmellose sodium, and optionally at least one further excipient, which at least one further excipient can be in particular selected from the group consisting of crospovidone, silica, magnesium stearate, povidone, and mixtures thereof.

The NSAID layer can be formed by compression of a mixture of the NSAID with other excipients. Preferably, the NSAID layer comprises granules comprising NSAID and at least one excipient. Preferably, the excipient in the NSAID granules is povidone and/or croscarmellose sodium. Preferably, the NSAID granules are formed by wet granulation.

The NSAID layer can comprise a composition comprising or consisting of granules and at least one optional pharmaceutically acceptable excipient, said granules comprising a non-steroidal anti-inflammatory drug (NSAID) and at least one pharmaceutically acceptable excipient, preferably said granules comprising NSAID being formed by wet granulation. The NSAID layer can be formed by compression of the composition comprising or consisting of granules and at least one optional pharmaceutically acceptable excipient.

The tablet core can be formed by one or more compression steps which may be chosen by the skilled person on the basis of the general knowledge in the art. Preferably, three layers can be compressed on a rotary tablet press in a three layer tableting mode to obtain a trilayer tablet core. Preferably, two layers can be compressed on a rotary tablet press in a bilayer tableting mode to obtain a bilayer tablet core. More generally, n layers (n being an integer which is at least 2, such as e.g. 2, 3, 4, 5, 6, ...) can be compressed on a rotary tablet press in a tableting mode for n layers to obtain a multilayer tablet core having n layers.

The term "PPI", as used herein, refers to a proton pump inhibitor which is a potent inhibitor of gastric acid secretion, inhibiting H⁺-K⁺-ATPase, the enzyme involved in the final step of hydrogen ion production in the parietal cells. The term includes, but is not limited to, the group consisting of pantoprazole, omeprazole, lansoprazole, rabeprazole and/or salts and enantiomers thereof. Preferably, the PPI is pantoprazole and/or pantoprazole sodium, most preferably, the PPI is pantoprazole sodium. The particle size of pantoprazole sodium is preferably below 150 *µ*m, more preferably below 100 *µ*m. Particle size determination is performed by laser diffraction method, using Malvern Mastersizer MS2000 equipped with Hydro 2000S dispersion unit, vegetable oil is used as dispersant.

The term "PPI layer", as used herein, refers to a layer in a multilayer tablet which comprises PPI. The PPI layer can in addition to multiple units of PPI comprise also additional excipient(s) selected from the group consisting of diluents, disintegrants, binders, stabilizers, glidants and/or lubricants. Preferably, the one or more additional excipients used in the PPI layer are preferably selected from the group consisting of mannitol, sorbitol, microcrystalline cellulose, crospovidone and/or calcium stearate. Most preferably, the additional excipient is microcrystalline cellulose. The PPI layer can comprise microcrystalline cellulose, and optionally one or more additional excipients, which can be preferably selected from the group consisting of mannitol, sorbitol, crospovidone, calcium stearate, and mixtures thereof.

The term "multiple units", as used herein, can in particular refer to (e.g two or more, or three or more, or ten or more) drug particles, granules, pellets, spheroids or mini tablets or similar units known to the skilled person. In case of using granules, their average particle size can be in the range of 40 to 400 *µ*m and in case of pellets average diameter of pellet cores can be in the range of 400 to 2,000 *µ*m. In case of using mini tablets, their maximum diameter can be in the range of 2 to 4 mm. Preferably, the multiple units of PPI comprise also a (or at least one) pharmaceutically acceptable excipient. Preferably, the (or the at least one) pharmaceutically acceptable excipient in the multiple units of PPI is selected from the group consisting of mannitol, sodium carbonate and/or crospovidone. A unit of PPI can comprise a proton pump inhibitor and optionally at least one pharmaceutically acceptable excipient, preferably the at least one pharmaceutically acceptable excipient can be selected from the group consisting of mannitol, sodium carbonate, crospovidone, and mixtures thereof.

The term "middle layer", as used herein, refers to a layer in the multilayer tablet present between the PPI layer and the NSAID layer. In case the NSAID layer contains acidic NSAID the middle layer provides additional protection of PPI. Middle layer can comprise at least one diluent and optional other excipients, in particular in a quantity which enables effective separation of NSAID and PPI containing layers. Effective separation is achieved when the maximum content of PPI individual degradation products in the solid composition stored packed in the primary packaging for 1 month at 40°C and 75% RH (relative air humidity) is less than 0.2%. In this paragraph the indication "%" refers to wt-%, based on the weight of the solid composition. The middle layer can extend over the complete surface of the NSAID layer facing the PPI layer.

The term "protective coating", as used herein, is intended to mean a layer of a polymeric or a non-polymeric material disposed on the surface of a particle core in order to avoid direct contact of the particle core with its environment. Protective coating preferably comprises water soluble polymer and optional other excipient(s) selected from plasticizers such as polyethylene glycol with average molecular weight from 200 to 8,000, triethyl citrate, 1,2-propylene glycol; pigments such as metal oxides selected from iron or titanium oxides; antitacking agents such as talc, magnesium stearate, glycerol monostearate; alkaline pH modifiers selected from inorganic alkaline reacting compounds selected from alkali and/or earth alkali metal oxides such as magnesium oxide, hydroxides such as magnesium hydroxide, sodium hydroxide, carbonates such as sodium carbonate, magnesium carbonate or calcium carbonate; organic alkaline reacting compounds such as ammonia, arginine, meglumine or the like. Polymers in protective coating can be selected from, but not limited to, water soluble cellulose ethers such as hydroxypropyl methylcellulose, propyl cellulose and/or hydroxyethyl cellulose; polyvinyl alcohol and its derivatives such as blockcopolymers of polyvinyl alcohol and polyethylene glycol commercially available from BASF as Kollicoat® IR or Kollicoat® Protect; and/or polyvinyl pyrrolidone (povidone) and/or copovidone. In a special embodiment of the invention protective coating can be selected from ready to use composition based on hydroxypropyl methylcellulose or polyvinyl alcohol obtainable under trade name Opadry, e.g. Opadry Clear OY-29020, Opadry II 85F190000 Clear, Opadry 03H28758 white, Opadry 33G28707 white, Opadry Y-1-7000 white, Opadry 85F28751 II HP white, Opadry 85F280010 II HP white, Opadry II white 31K58875, Opadry AMB white OY-B-28920. The protective coating is applied onto the multiple units of PPI in the quantity to achieve weight gain in the range of 5 to 20w/w%, preferably 7 to 15 w/w% and most preferably 8 to 12w/w%. Preferably, the protective coating comprises hydroxypropyl methylcellulose, povidone and/or sodium carbonate.

The term "barrier coating", as used herein, refers to a coating surrounding the multilayer tablet and is present between the multilayer tablet and gastro-resistant coating. Barrier coating additionally protects the PPI in the core from the acidic gastro-resistant coating. The barrier coating is preferably composed from at least one water soluble polymer, which can be selected from the group consisting of water soluble cellulose ethers such as hydroxypropyl methylcellulose, propyl cellulose and/or hydroxyethyl cellulose; polyvinyl alcohol and its derivatives such as blockcopolymers of polyvinyl alcohol and polyethylene glycol commercially available from BASF as Kollicoat® IR or Kollicoat® Protect; and/or polyvinyl pyrrolidone (povidone) and/or copovidone and at least one further excipient selected from plasticizers, antitacking agents, alkaline pH modifiers, pigments and colorants which can be the same as those comprised in protective coating. Preferably, the barrier coating comprises a polymer selected from the group consisting of hypromellose, povidone and/or polyethylene glycol. Most preferably, the barrier coating comprises hypromellose. The thickness of the barrier coating is preferably in the range of 20 to 100 *µ*m, preferably 25 to 80 *µ*m and most preferably 30 to 60 *µ*m.

The term "gastro-resistant coating", as used herein, is interchangeable with the term "enteric coating" and it refers to a coating surrounding the barrier coating, the solubility of the coating being dependent on the pH, in particular in such a manner that it prevents the release of the drug in the stomach but permits the release of the drug at some stage after the formulation has emptied from the stomach. Gastro-resistant coating can comprise at least one polymer being insoluble in aqueous solutions having pH value of less than 4.5 and at least one further excipient selected from plasticizers, antitacking agents, pigments, colorants and/or surface active substances. The thickness of the gastro-resistant coating is preferably in the range of 40 to 250 *µ*m, preferably 45 to 200 *µ*m and most preferably 50 to 180 *µ*m.
Polymer(s) being insoluble in aqueous solutions having pH value of less than 4.5 can be selected from the group consisting of cellulose esters such as cellulose acetate phthalate, hydroxypropyl methylcellulose acetate succinate or cellulose acetate succinate; and/or methacrylic acid copolymers such as those sold as Eudragit® L, Eudragit® S by Evonik or Acryleze® by Colorcon. Preferably, the polymer is hydroxypropyl methylcellulose acetate succinate and/or methacrylic acid - ethyl acrylate copolymer. Most preferably, the polymer in the gastro-resistant coating is methacrylic acid - ethyl acrylate copolymer (1:1).
Plasticizer(s) can be selected from, but not limited to, the group consisting of polyethylene glycol with average molecular weight from 200 to 8.000, 1,2-propylene glycol, triethyl citrate, acetyl triethyl citrate, polysorbate 80 and/or dibutyl sebacate. Preferably, the plasticizer in the gastro-resistant coating is triethyl citrate.

The thickness of the gastro-resistant coating and barrier coating can be determined by the following method. The tablet is cross-sected and analysed using a scanning electron microscope (SEM) especially with appropriate software to determine the thickness of the coating. The thickness of the coating is measured on different parts of the tablet surface, where the number and position of measuring points depend on the size and shape of the coated tablet. For example, in case of concave shape of coated tablet, three measurements are performed on both sides of the concave tablet (in centre and on both sides) ; the thickness is also determined on all four edges of the tablet and also at the centre of the edge of the tablet. The analysis is carried out on at least three tablets. The average result of all measurements is considered as the final result.

Diluent(s) can be selected from water soluble diluents such as mono, di and/or oligosaccharides like lactose, sucrose, dextran; sugar alcohols such as mannitol, sorbitol, maltitol and the like; water insoluble diluents such as cellulose subtypes like cellulose powder, microcrystalline cellulose, silicified microcrystalline cellulose and other co-processed types of microcrystalline cellulose, starch and starch derivatives and subtypes like corn starch, pregelatinized starch, inorganic calcium salts such as calcium hydrogen phosphate in anhydrous or hydrated form. Diluent(s) used in extragranular phase are preferably of types for direct compression (DC) meaning that their particles are engineered in order to achieve better flowability and/or compressibility and/or compactability such types obtained by agglomeration, granulation, spherical crystallization, spray drying, co-crystallization or similar processes. Typical extragranular diluents are spray dried mannitol and/or sorbitol, granulated lactose, mannitol and/or sorbitol, silicified microcrystalline cellulose and/or mixtures thereof.

Binder(s) can be selected from cellulose ethers such as hydroxypropyl cellulose (HPC), hydroxypropyl methylcellulose (HPMC), specially preferred HPMC type is 2910 according to US Pharmacopoeia (USP) with viscosity of its 2 w/vol% aqueous solutions in the range of 2 to 20 mPas, preferably 3 to 15mPas, and hydroxyethyl cellulose (HEC), povidone with K value in the range of 10 to 100, preferably 20 to 40, copovidone and combinations thereof.

Disintegrant(s) can be selected from crospovidone, croscarmellose sodium or calcium, carmellose sodium or calcium, low substituted hydroxylpropyl cellulose, sodium starch glycolate, salts of carboxymethyl cellulose, salts of polacrilin or mixtures thereof. Croscarmellose sodium and crospovidone being preferred disintegrants.

Examples of pharmaceutically accepted glidants are talc, silica such as colloidal and hydrophobic colloidal silica, magnesium oxide, powdered cellulose, silicates such as magnesium silicate, magnesium trisilicate etc. Glidant may be present in the amount from 0.1% to 5% by weight of the composition.

Lubricant(s) can be selected from stearic acid, a metal salt of stearic acid such as magnesium stearate, talc, colloidal silica, a wax variety such as beads wax, spermaceti, boric acid, adipic acid, sodium sulphate, fumaric acid, stearyl sodium fumarate, sucrose aliphatic acid ester, a lauryl sulphate such as sodium lauryl sulphate, magnesium lauryl sulphate, starch derivative such as corn starch, potato starch, glyceryl behenate, behenoyl polyoxyl glyceride etc. Magnesium stearate and/or calcium stearate are being preferred.

Stabilizer(s) can be selected from pharmaceutically acceptable alkaline reacting inorganic and/or organic compounds such as metal oxides like magnesium oxide, metal carbonates like sodium, magnesium and/or calcium carbonate, metal phosphates like disodium and/or trisodium phosphate, hydroxides like magnesium or calcium hydroxide, oxides like magnesium or calcium oxide, alkaline reacting amino acids like arginine, low molecular amines like meglumine or the mixture(s) thereof.

Controlled release excipient(s) can be selected in one embodiment from pharmaceutically acceptable hydrophilic polymer substances, in particular having average molecular weight of more than 1kDa being able to swell and form a gel structure in contact with aqueous media. Examples of hydrophilic polymers are known to the professional artisan. Most preferred examples are hydroxypropyl methylcellulose having viscosity of their 2w/vol% aqueous dispersion of more than 50mPas at room temperature, preferably of more than 1,000 mPas and most preferably of more than 3,000 mPas, polyethylene oxide having average molecular weight of at least 500,000, methacrylic ester copolymers like Eudragit® NE, carbomers having viscosity of 0.5 w/vol% aqueous dispersion in the range from 3,000 to 50,000 mPas (Brookfield RVT Viscosity, at pH 7.5, 20 rpm at 25°C), natural polymers such as alginic acid salts, carragenanes, xanthanes, gums such as locust bean gum, or the like and/or the mixture(s) thereof. In a preferred embodiment particles of controlled release excipients are at least partially in intimate contact with particles of NSAID, which can be achieved by state of the art granulation of NSAID and at least one controlled release excipients together with optional other excipients selected from diluents, binders and/or pH modifiers. In another embodiment controlled release excipients can be selected from pharmaceutically acceptable substances being insoluble in water and having no or low ability to swell and form a gel structures in aqueous media such as, but not limited to, alkyl cellulose ethers like ethylcellulose, cellulose acetate, copolymers of ammonium methacrylate copolymers obtainable as Eudragit® RL and/or Eudragit® RS, polyvinyl acetate alone or admixed with povidone obtainable as Kollidon® SR.

Antitacking agent can be selected from talc, magnesium stearate, glycerol monostearate and/or colloidal silicon dioxide.

The multilayer tablet core may comprise a NSAID layer, wherein the NSAID is or comprises naproxen or a salt thereof, and a PPI layer, wherein the PPI is or comprises pantoprazole or a salt thereof.

In particular, the present invention relates to a pharmaceutical tablet composition comprising:
a) a multilayer tablet core comprising:
   i) a non-steroidal anti-inflammatory drug (NSAID) layer comprising NSAID (preferably naproxen or a salt thereof) and at least one pharmaceutically acceptable excipient, the NSAID layer comprising granules, which granules comprise NSAID and at least one pharmaceutically acceptable excipient,
   ii) an optional middle layer,
   iii) a proton pump inhibitor (PPI) layer comprising multiple units of PPI coated with a protective coating, preferably the PPI being pantoprazole or a salt thereof,
b) barrier coating surrounding the multilayer tablet core; and
c) gastro-resistant coating surrounding the barrier coating.

In the following, the present invention will be illustrated with several non-limiting examples.

### EXAMPLES

### Example 1: Naproxen granules

| Ingredients | Amount [mg/tablet] |
|---|---|
| Naproxen | 500.00 |
| Povidone K25 | 10.00 |
| Croscarmellose Sodium | 10.50 |
| Total mass of Naproxen granules | 520.50 |

### Manufacturing procedure:

The active ingredient was mixed with croscarmellose sodium in powder form. Then, the granulation liquid consisting of povidon K25 solution in purified water was sprayed onto the obtained powder mixture and granulating/kneading was performed using a high-shear granulator. Afterwards, drying and sieving was performed to obtain final naproxen granules. Loss on drying (LOD) of final granules was ≤1.0% determined by IR halogen dryer e.g. Mettler HR 73 type at 105°C for 5 min.

### Examples 2-3 and Comparative example 1: Coated pantoprazole granules and pantoprazole granules

| Ingredients | Amount [mg/tablet] | | |
|---|---|---|---|
| | Comp. ex. 1 | Ex. 2 | Ex. 3 |
| Granules | | | |
| Pantoprazole Sodium Sesquihydrate | 11.28 | 11.28 | 11.28 |
| Mannitol | 3.38 | 3.38 | 3.38 |
| Sodium Carbonate, Anhydrous | 2.50 | 2.50 | 2.50 |
| Crospovidone | 3.75 | 3.75 | 3.75 |
| Total mass of Pantoprazole granules | 20.91 | 20.91 | 20.91 |

| Coated granules | | | |
|---|---|---|---|
| Hypromellose 3 cP | / | 2.35 | / |
| Povidone K12 | | / | 2.35 |
| Sodium Carbonate, Anhydrous | / | 0.06 | 0.06 |
| Total mass of coated Pantoprazole granules | / | 23.32 | 23.32 |

### Manufacturing procedure:

The active ingredient was mixed with mannitol, sodium carbonate, anhydrous and crospovidone in powder form. Then, the granulation liquid consisting of purified water and optionally a part of sodium carbonate, anhydrous was sprayed onto the powder mixture and granulating/kneading was performed using a high-shear granulator. Afterwards, drying and sieving was performed to obtain final pantoprazole granules. Furthermore, in case of examples 2-3 the obtained granules were coated with water solution of hypromellose 3 cP or povidone K12 and anhydrous sodium carbonate using fluid-bed coater. Loss on drying (LOD) of final granules and final coated granules was ≤1.0% determined by IR halogen dryer e.g. Mettler HR 73 type at 105°C for 5 min.

### Examples 4-6 and Comparative example 2: Bilayer tablets

| Ingredients | Amount [mg/tablet] | | | |
|---|---|---|---|---|
| | Comp. ex. 2 | Ex. 4 | Ex. 5 | Ex. 6 |
| 1. layer | | | | |
| Naproxen granules* | 520.50 | 520.50 | 520.50 | 520.50 |
| Croscarmellose Sodium | 10.50 | 10.50 | 10.50 | 10.50 |
| Iron Oxide, Yellow | 0.50 | 0.50 | 0.50 | 0.50 |
| Silica, Colloidal Anhydrous | 3.10 | 3.10 | 3.10 | 3.10 |
| Magnesium stearate | 5.40 | 5.40 | 5.40 | 5.40 |
| Total mass of 1. layer | 540.00 | 540.00 | 540.00 | 540.00 |

| 2. layer | | | | |
|---|---|---|---|---|
| Pantoprazole granules** | 20.91 | / | / | / |
| Hypromellose based coated Pantoprazole granules*** | / | 23.32 | 23.32 | / |
| Povidone based coated Pantoprazole granules**** | / | / | / | 23.32 |
| Mannitol DC | 138.89 | 136.48 | / | 136.48 |
| Sorbitol DC | / | / | 80.00 | / |
| Cellulose, Microcrystalline, type 200 | / | 80.00 | / | / |
| Cellulose, Microcrystalline, type 112 | 80.00 | / | 136.48 | 80.00 |
| Crospovidone | 27.00 | 27.00 | 27.00 | 27.00 |
| Calcium stearate | 3.20 | 3.20 | 3.20 | 3.20 |
| Total mass of 2. layer | 270.00 | 270.00 | 270.00 | 270.00 |

| Barrier film coating | | | | |
|---|---|---|---|---|
| Hypromellose 3 cP | 20.95 | 20.95 | 21.47 | 21.47 g |
| Povidone K25 | 0.52 | 0.52 | / | / |
| Titanium Dioxide | 0.38 | 0.38 | 0.38 | 0.38 |
| Propylene glycol | 5.15 | 5.15 | 5.15 | 5.15 |
| Total mass of barrier coat | 27.00 | 27.00 | 27.00 | 27.00 |

| Gastro-resistant coating | | | | |
|---|---|---|---|---|
| Methacrylic Acid - Ethyl Acrylate Copolymer (1:1) dispersion 30 per cent¹ | 59.28 | 59.28 | 59.28 | 59.28 |
| Talc | 14.80 | 14.80 | 14.80 | 14.80 |
| Triethyl Citrate | 5.92 | 5.92 | 5.92 | 5.92 |
| Total mass of gastro-resistant coat | 80.00 | 80.00 | 80.00 | 80.00 |

| | | | | |
|---|---|---|---|---|
| *Naproxen granules (corresponding to 500.00 mg of naproxen) consist of: naproxen, povidone K25 and croscarmellose sodium (as described in more detail in Ex. 1). **Pantoprazole granules (corresponding to 10.00 mg of pantoprazole) consist of: pantoprazole sodium sesquihydrate, mannitol, anhydrous sodium carbonate and crospovidone (as described in more detail in Comparative Example 1). *** Hypromellose based coated pantoprazole granules consist of: pantoprazole granules**, hypromellose 3 cP and anhydrous sodium carbonate (as described in more detail in Example 2). ****Povidone based coated pantoprazole granules: pantoprazole granules**, povidone K12 and anhydrous sodium carbonate (as described in more detail in Example 3). ¹Expressed as a weight of dry solids incorporated into Eudragit L30D 30% aqueous dispersion. | | | | |

### Manufacturing procedure:

The obtained naproxen granules were mixed with croscarmellose sodium, iron oxide, yellow, silica, colloidal anhydrous and magnesium stearate to form final composition ready for tableting.

The obtained pantoprazole granules or hypromellose based coated Pantoprazole granules or povidone based coated pantoprazole granules were mixed with mannitol DC or sorbitol DC, cellulose, microcrystalline, crospovidone and calcium stearate to form final composition ready for tableting.

Bilayer tablets containing naproxen and pantoprazole were prepared by first introducing the naproxen containing mixture ready for tabletting into the tabletting machine (1.layer), followed by the pantoprazole containing mixture ready for tabletting (2. layer). These two layers were then compressed on a rotary tablet press in a bilayer tabletting mode.

Then, hypromellose 3 cP and optionally povidone K25, titanium dioxide and propylene glycol were dispersed in purified water. Bilayer tablet cores were coated in a conventional coating pan with the obtained dispersion to form barrier film coating. The thickness of the barrier coating was 44 *µ*m.

Finally, talc and triethyl citrate are homogenized in water using a homogenizer (e.g. Ultra Turrax. The excipient suspension was poured slowly into the methacrylic acid - ethyl acrylate copolymer (1:1) dispersion 30 per cent while stirring gently with a conventional stirrer. Barrier film coated tablets were coated in a conventional coating pan with the obtained dispersion to form gastro-resistant coat, i.e. final gastro-resistant tablets. The thickness of the gastro-resistant coating is 100 *µ*m and 103 *µ*m for Ex.5 and Ex. 6, respectively.

### Comparative example 3: Monolayer tables

| Ingredients | Amount [mg/tablet] |
|---|---|
| Tablet core | |
| Naproxen granules* | 520.50 |
| Hypromellose based coated Pantoprazole granules*** | 23.32 |
| Croscarmellose Sodium | 16.00 |
| Silica, Colloidal Anhydrous | 4.78 |
| Magnesium stearate | 5.90 |
| Total mass of tablet core | 590.00 |

| Barrier film coating | |
|---|---|
| Hypromellose 3 cP | 19.40 |
| Povidone K25 | 0.48 |
| Titanium Dioxide | 0.35 |
| Propylene glycol | 4.77 |
| Total mass of barrier coat | 25.00 |

| Gastro-resistant coating | |
|---|---|
| Methacrylic Acid - Ethyl Acrylate Copolymer (1:1) dispersion 30 per cent¹ | 51.60 |
| Talc | 13.00 |
| Triethyl Citrate | 5.40 |
| Total mass of gastro-resistant coat | 70.00 |

| | |
|---|---|
| *Naproxen granules (corresponding to 500.00 mg of naproxen) consist of: naproxen, povidone K25 and croscarmellose sodium (as described in more detail in Example 1). **Pantoprazole granules (corresponding to 10.00 mg of pantoprazole) consist of: pantoprazole sodium sesquihydrate, mannitol, anhydrous sodium carbonate and crospovidone (as described in more detail in Comparative Example 1). ***Hypromellose based coated pantoprazole granules consist of: pantoprazole granules**, hypromellose 3 cP and anhydrous sodium carbonate (as described in more detail in Example 2). ¹Expressed as a weight of dry solids incorporated into Eudragit L30D 30% aqueous dispersion. | |

### Manufacturing procedure:

The obtained naproxen granules and hypromellose based coated pantoprazole granules were mixed with croscarmellose sodium, silica, colloidal anhydrous and magnesium stearate to form final composition ready for tableting using a rotary tablet press in conventional mono layer tableting mode.

Then, hypromellose 3 cP, povidone K25, titanium dioxide and propylene glycol were dispersed in purified water. Tablet cores comprising naproxen and pantoprazole were coated in a conventional coating pan with the obtained dispersion to form barrier film coat.

Finally, talc and triethyl citrate are homogenized in water using a homogenizer (e.g. Ultra Turrax). The excipient suspension was poured slowly into the methacrylic acid - ethyl acrylate copolymer (1:1) dispersion 30 per cent while stirring gently with a conventional stirrer. Barrier film coated tablets were coated in a conventional coating pan with the obtained dispersion to form gastro-resistant coat, i.e. final gastro-resistant tablets.

### Examples 7-8: Trilayer tablets

| Ingredients | Amount [mg/tablet] | |
|---|---|---|
| | Ex. 7 | Ex. 8 |
| 1. layer | | |
| Naproxen granules* | 520.50 | 520.50 |
| Croscarmellose Sodium | 10.50 | 10.50 |
| iron Oxide, Yellow | 0.50 | 0.50 |
| Silica, Colloidal Anhydrous | 3.10 | 3.10 |
| Magnesium stearate | 5.40 | 5.40 |
| Total mass of 1. layer | 540.00 | 540.00 |

| 2. layer (middle layer) | | |
|---|---|---|
| Cellulose, Microcrystalline, type 112 | 63.00 | 63.00 |
| Manitol DC | 36.00 | 36.00 |
| Magnesium stearate | 1.00 | 1.00 |
| Total mass of 2. layer | 100.00 | 100.00 |

| 3. layer | | |
|---|---|---|
| Hypromellose based coated pantoprazole granules*** | 23.32 | 23.32 |
| Mannitol DC | 136.48 | 80.00 |
| Cellulose, Microcrystalline, type 200 | / | 136.48 |
| Cellulose, Microcrystalline, type 112 | 80.00 | / |
| Crospovidone | 27.00 | 27.00 |
| Calcium stearate | 3.20 | 3.20 |
| Total mass of 3. layer | 270.00 | 270.00 |

| Barrier film coating | | |
|---|---|---|
| Hypromellose 3 cP | 20.95 | 20.95 |
| Povidone K25 | 0.52 | 0.52 |
| Titanium Dioxide | 0.38 | 0.38 |
| Propylene glycol | 5.15 | 5.15 |
| Total mass of barrier coating | 27.00 | 27.00 |

| Gastro-resistant coating | | |
|---|---|---|
| Methacrylic Acid - Ethyl Acrylate | 59.28 | 59.28 |
| Copolymer (1:1) dispersion 30 per cent¹ | | |
| Talc | 14.80 | 14.80 |
| Triethyl Citrate | 5.92 | 5.92 |
| Total mass of gastro-resistant coat | 80.00 | 80.00 |

| | | |
|---|---|---|
| *Naproxen granules (corresponding to 500.00 mg of naproxen) consist of: naproxen, povidone K25 and croscarmellose sodium (as described in more detail in Example 1). ***Hypromellose based coated pantoprazole granules consist of: pantoprazole granules**, hypromellose 3 cP and anhydrous sodium carbonate (as described in more detail in Example 2). ¹Expressed as a weight of dry solids incorporated into Eudragit L30D 30% aqueous dispersion. | | |

### Manufacturing procedure:

The obtained naproxen granules were mixed with croscarmellose sodium, iron oxide, yellow, silica, colloidal anhydrous and magnesium stearate to form final composition ready for tableting. The obtained hypromellose based coated pantoprazole granules were mixed with mannitol DC, microcrystalline cellulose, crospovidone and calcium stearate to form final composition ready for tableting.

Middle layer was prepared by mixing cellulose, microcrystalline, mannitol DC and magnesium stearate in a tumbling mixer in order to obtain a final composition ready for tableting.

Trilayer tablets comprising naproxen and pantoprazole were prepared by first introducing the naproxen containing mixture ready for tableting into the tableting machine (1.layer). Then, the middle layer (2.layer) was introduced, followed by the pantoprazole containing mixture ready for tableting (3. layer). These three layers were then compressed on a rotary tablet press in a three layer tableting mode.

Then, hypromellose 3 cP and povidone K25, titanium dioxide and propylene glycol were dispersed in purified water. Trilayer tablet cores were coated in a conventional coating pan with the obtained dispersion to form barrier film coating.

Finally, talc and triethyl citrate are homogenized in water using a homogenizer (e.g. Ultra Turrax. The excipient suspension was poured slowly into the methacrylic acid - ethyl acrylate copolymer (1:1) dispersion 30 per cent while stirring gently with a conventional stirrer. Barrier film coated tablets were coated in a conventional coating pan with the obtained dispersion to form gastro-resistant coat, i.e. final gastro-resistant tablets.

### Comparative example 4: Monolayer tablets according to the VIMOVO modified-release tablets

| ingredients | Amount [mg/tablet] |
|---|---|
| Tablet core | |
| Naproxen granules* | 520.50 |
| Croscarmellose Sodium | 10.50 |
| Iron Oxide, Yellow | 0.50 |
| Silica, Colloidal Anhydrous | 3.10 |
| Magnesium stearate | 5.40 |
| Total mass of tablet core | 540.00 |

| Gastro-resistant coating | |
|---|---|
| Methacrylic Acid - Ethyl Acrylate Copolymer (1:1) dispersion 30 per cent¹ | 51.60 |
| Talc | 13.00 |
| Triethyl Citrate | 5.40 |
| Total mass of gastro-resistant coating | 70.00 |

| Barrier coating | |
|---|---|
| Opadry Clear OY-29020 | 20.00 |
| Total mass of barrier coating | 20.00 |

| Pantoprazole coating | |
|---|---|
| Pantoprazole Sodium Sesquihydrate | 22.55 |
| Sodium Carbonate, Anhydrous | 7.50 |
| Opadry Clear OY-29020¹ | 22.45 |
| Total mass of pantoprazole coating | 52.50 |

| Protective coating | |
|---|---|
| Opadry 85F28751 II HP white² | 20.00 |
| Total mass of protective coating | 20.00 |

| | |
|---|---|
| ¹Opadry Clear OY-29020 is ready-to-use dry mixture of hypromellose and macrogol/PEG. ²Opadry 85F28751 II HP white is ready-to-use dry mixture of polyvinyl alcohol-part. Hydrolyzed, titanium dioxide, macrogol/PEG and talc. | |

### Manufacturing procedure:

The obtained naproxen granules were mixed with croscarmellose sodium, iron oxide, yellow, silica, colloidal anhydrous and magnesium stearate to form final composition ready for tableting using a rotary tablet press in conventional mono layer tableting mode.

Then, talc and triethyl citrate are homogenized in water using a homogenizer (e.g. Ultra Turrax). The excipient suspension was poured slowly into the methacrylic acid - ethyl acrylate copolymer (1:1) dispersion 30 per cent while stirring gently with a conventional stirrer. Tablet cores containing naproxen were coated in a conventional coating pan with the obtained dispersion to form gastro-resistant coating.

Opadry Clear OY-29020 is added slowly to purified water. The dispersion is sprayed on the obtained gastro-resistant tablets in a conventional coating pan in order to form barrier coating.

Afterwards, pantoprazole sodium sesquihydrate is dissolved in purified water containing sodium carbonate in solution. After mixing, Opadry Clear OY-29020 is added and mixing is continued until Opadry is fully dispersed. The dispersion is sprayed on the obtained barrier coated tablets in a conventional coating pan in order to form pantoprazol coating.

Finally, Opadry 85F28751 II HP white is added slowly to purified water. The dispersion is sprayed on the obtained pantoprazole coated tablets in a conventional coating pan in order to form final protective coat.

**Example 9:** Comparison of stress stability of formulations according to the present invention and comparative examples: tablets were packed in closed vials and stored 1 month at 40°C (impurities were determined by HPLC chromatography). The method used for the determination of related substances of pantoprazole and naproxen was a reversed-phase gradient HPLC method, external standard method, using a 3.5 *µ*m C8 column, or suitably validated alternative. The mobile phase was a mixture of 0.02 M ammonium acetate and acetonitrile, with UV detection at 290 nm.

| | Maximum individual impurity of pantoprazole (%) | Total impurities of pantoprazole (%) |
|---|---|---|
| Comparative example 2 | 0.40 | 1.42 |
| Comparative example 3 | 0.27 | 0.85 |
| Comparative example 4 | 0.33 | 0.79 |
| Example 4 | 0.17 | 0.58 |

As evident from the stability data in the table above, the stability of pantoprazole in the pharmaceutical tablet composition of the present invention is superior to stability of pantoprazole in formulations without protective coating and superior to stability of formulation comprising pantoprazole with protective coating and naproxen in a single layer.

### Examples 10-12: Bilayer tablets with Pantoprazole

| Ingredients | Amount [mg/tablet] | | |
|---|---|---|---|
| | Example. 10 | Ex. 11 | Ex. 12 |
| 1. layer | | | |
| Naproxen granules* | 520.50 | 520.50 | 520.50 |
| Croscarmellose Sodium | 10.50 | 10.50 | 10.50 |
| Iron Oxide, Yellow | 0.50 | 0.50 | 0.50 |
| Silica, Colloidal Anhydrous | 3.10 | 3.10 | 3.10 |
| Magnesium stearate | 5.40 | 5.40 | 5.40 |
| Total mass of 1. layer | 540.00 | 540.00 | 540.00 |

| 2. layer | | | |
|---|---|---|---|
| Hypromellose based coated Pantoprazole granules*** | 23.32 | 23.32 | 23.32 |
| Methacrylic Acid - Ethyl Acrylate Copolymer (1:1) dispersion 30 per cent¹ | 0.69 | 1.38 | 2.07 |
| Talc | 0.17 | 0.35 | 0.52 |
| Triethyl Citrate | 0.07 | 0.14 | 0.21 |
| Total mass of Pantoprazole granules coated with Hypromellose and Gastro-resistant coating | 24.25 | 25.19 | 26.12 |
| Cellulose, Microcrystalline, type 200 | 135.55 | 134.61 | 133.68 |
| Mannitol DC | 80.00 | 80.00 | 80.00 |
| Crospovidone | 27.00 | 27.00 | 27.00 |
| Calcium stearate | 3.20 | 3.20 | 3.20 |
| Total mass of 2. layer | 270.00 | 270.00 | 270.00 |

| Barrier film coating | | | |
|---|---|---|---|
| Hypromellose 3 cP | 20.95 | 20.95 | 21.47 |
| Povidone K25 | 0.52 | 0.52 | / |
| Titanium Dioxide | 0.38 | 0.38 | 0.38 |
| Propylene glycol | 5.15 | 5.15 | 5.15 |
| Total mass of barrier coat | 27.00 | 27.00 | 27.00 |

| Gastro-resistant coating | | | |
|---|---|---|---|
| Methacrylic Acid - Ethyl Acrylate | 59.28 | 59.28 | 59.28 |
| Copolymer (1:1)dispersion 30 per cent¹ | | | |
| Talc | 14.80 | 14.80 | 14.80 |
| Triethyl Citrate | 5.92 | 5.92 | 5.92 |
| Total mass of gastro-resistant coat | 80.00 | 80.00 | 80.00 |

| | | | |
|---|---|---|---|
| *Naproxen granules (corresponding to 500.00 mg of naproxen) consist of: naproxen, povidone K25 and croscarmellose sodium (as described in more detail in Ex. 1). **Pantoprazole granules (corresponding to 10.00 mg of pantoprazole) consist of: pantoprazole sodium sesquihydrate, mannitol, anhydrous sodium carbonate and crospovidone (as described in more detail in Comparative Example 1). ***Hypromellose based coated pantoprazole granules consist of: pantoprazole granules**, hypromellose 3 cP and anhydrous sodium carbonate (as described in more detail in Example 2). ¹Expressed as a weight of dry solids incorporated into Eudragit L30D 30% aqueous dispersion. | | | |

### Manufacturing procedure:

The obtained naproxen granules were mixed with croscarmellose sodium, iron oxide, yellow, silica, colloidal anhydrous and magnesium stearate to form final composition ready for tableting.

Talc and triethyl citrate are homogenized in water using a homogenizer (e.g. Ultra Turrax. The excipient suspension was poured slowly into the methacrylic acid - ethyl acrylate copolymer (1:1) dispersion 30 per cent while stirring gently with a conventional stirrer. Hypromellose based coated pantoprazole granules were coated in a conventional coating pan with the obtained dispersion to form gastro-resistant coat, i.e. pantoprazole granules coated with hypromellose and gastro-resistant coating. The obtained pantoprazole granules coated with hypromellose and gastro-resistant coating were mixed with mannitol DC, microcrystalline cellulose, crospovidone and calcium stearate to form final composition ready for tableting.

Bilayer tablets containing naproxen and pantoprazole were prepared by first introducing the naproxen containing mixture ready for tabletting into the tabletting machine (1.layer), followed by the pantoprazole containing mixture ready for tabletting (2. layer). These two layers were then compressed on a rotary tablet press in a bilayer tabletting mode.

Then, hypromellose 3 cP and optionally povidone K25, titanium dioxide and propylene glycol were dispersed in purified water. Bilayer tablet cores were coated in a conventional coating pan with the obtained dispersion to form barrier film coating.

Finally, talc and triethyl citrate are homogenized in water using a homogenizer (e.g. Ultra Turrax. The excipient suspension was poured slowly into the methacrylic acid - ethyl acrylate copolymer (1:1) dispersion 30 per cent while stirring gently with a conventional stirrer. Barrier film coated tablets were coated in a conventional coating pan with the obtained dispersion to form gastro-resistant coat, i.e. final gastro-resistant tablets.

### Example 13: Bilayer tablet with esomeprazole

| Ingredients | Amount [mg/tablet] |
|---|---|
| | Example. 13 |
| 1. layer | |
| Naproxen granules* | 520.50 |
| Croscarmellose Sodium | 10.50 |
| Iron Oxide, Yellow | 0.50 |
| Silica, Colloidal Anhydrous | 3.10 |
| Magnesium stearate | 5.40 |
| Total mass of 1. layer | 540.00 |

| 2. layer | |
|---|---|
| Esomeprazole Magnesium Dihydrate | 10.84 |
| Mannitol | 3.82 |
| Sodium Carbonate, Anhydrous | 2.50 |
| Crospovidone | 3.75 |
| Hypromellose 3 cP | 2.35 |
| Sodium Carbonate, Anhydrous | 0.06 |
| Total mass of Esomeprazole granules coated with Hypromellose coating | 23.32 |
| Methacrylic Acid - Ethyl Acrylate Copolymer (1:1) dispersion 30 per cent¹ | 1.38 |
| Talc | 0.35 |
| Triethyl Citrate | 0.14 |
| Total mass of Esomeprazole granules coated with Hypromellose and Gastro-resistant coating | 25.19 |
| Cellulose, Microcrystalline, type 200 | 134.61 |
| Mannitol DC | 80.00 |
| Crospovidone | 27.00 |
| Calcium stearate | 3.20 |
| Total mass of 2. layer | 270.00 |

| Barrier film coating | |
|---|---|
| Hypromellose 3 cP | 20.95 |
| Povidone K25 | 0.52 |
| Titanium Dioxide | 0.38 |
| Propylene glycol | 5.15 |
| Total mass of barrier coat | 27.00 |

| Gastro-resistant coating | |
|---|---|
| Methacrylic Acid - Ethyl Acrylate Copolymer (1:1) dispersion 30 per cent¹ | 59.28 |
| Talc | 14.80 |
| Triethyl Citrate | 5.92 |
| Total mass of gastro-resistant coat | 80.00 |

| | |
|---|---|
| *Naproxen granules (corresponding to 500.00 mg of naproxen) consist of: naproxen, povidone K25 and croscarmellose sodium (as described in more detail in Ex. 1). ¹Expressed as a weight of dry solids incorporated into Eudragit L30D 30% aqueous dispersion. | |

### Manufacturing procedure:

Bilayer tablets were prepared according to Example 11, except that pantoprazole sodium sesquihydrate and sodium carbonate, anhydrous were substituted with esomeprazole magnesium dihydrate and magnesium hydroxide carbonate heavy respectively.

## Claims

1. A pharmaceutical tablet composition comprising:
a) a multilayer tablet core comprising:
i) a non-steroidal anti-inflammatory drug (NSAID) layer comprising NSAID and at least one pharmaceutically acceptable excipient;
ii) an optional middle layer;
iii) a proton pump inhibitor (PPI) layer comprising multiple units of PPI coated with a protective coating;
b) barrier coating surrounding the multilayer tablet core; and
c) gastro-resistant coating surrounding the barrier coating.

2. The pharmaceutical tablet composition according to claim 1, wherein the NSAID is in a free acid form.

3. The pharmaceutical tablet composition according to claim 1, wherein the NSAID is selected from the group consisting of naproxen, ibuprofen, diclofenac, acetylsalicylic acid and/or salts and enantiomers thereof.

4. The pharmaceutical tablet composition according to claim 3, wherein the NSAID is selected from the group consisting of naproxen, ibuprofen, diclofenac and/or enantiomers thereof.

5. The pharmaceutical tablet composition according to claim 3, wherein the NSAID is naproxen and/or naproxen sodium.

6. The pharmaceutical tablet composition according to claim 4 or 5, wherein the NSAID is naproxen.

7. The pharmaceutical tablet composition according to any one of the preceding claims, wherein the PPI is selected from the group consisting of pantoprazole, omeprazole, lansoprazole and/or salts and enantiomers thereof.

8. The pharmaceutical tablet composition according to claim 7, wherein the PPI is pantoprazole and/or pantoprazole sodium.

9. The pharmaceutical tablet composition according to any one of the preceding claims, wherein the NSAID layer comprises pharmaceutically acceptable excipient selected from the group consisting of croscarmellose sodium, crospovidone, silica, magnesium stearate, and/or povidone.

10. The pharmaceutical tablet composition according to any one of the preceding claims, wherein the NSAID layer comprises granules comprising NSAID and at least one pharmaceutically acceptable excipient.

11. The pharmaceutical tablet composition according to any one of the preceding claims, wherein the PPI layer in addition to multiple units of PPI comprises additional excipient(s) selected from the group consisting of mannitol, sorbitol, microcrystalline cellulose, crospovidone and/or calcium stearate.

12. The pharmaceutical tablet composition according to any one of the preceding claims, wherein multiple units of PPI comprise a pharmaceutically acceptable excipient which is selected from the group consisting of mannitol, sodium carbonate and/or crospovidone.

13. The pharmaceutical tablet composition according to any one of the preceding claims, wherein the protective coating comprises hydroxypropyl methylcellulose, povidone and/or sodium carbonate.

14. The pharmaceutical tablet composition according to any one of the preceding claims, wherein the barrier coating comprises a polymer selected from the group consisting of hypromellose, povidone and/or polyethylene glycol.

15. The pharmaceutical tablet composition according to any one of the preceding claims, wherein the gastro-resistant coating comprises hydroxypropyl methylcellulose acetate and/or methacrylic acid - ethyl acrylate copolymer.

## Patentansprüche

1. Pharmazeutische Tablettenzusammensetzung, umfassend:
a) einen Mehrschicht-Tablettenkern, umfassend:
i) eine nicht-steroidaler entzündungshemmender Arzneistoff (NSAID)-Schicht, umfassend NSAID und mindestens einen pharmazeutisch verträglichen Exzipienten;
ii) eine optionale mittlere Schicht;
iii) eine Protonenpumpeninhibitor (PPI)-Schicht, umfassend multiple Einheiten von PPI, überzogen mit einem Schutzüberzug;
b) Barriereüberzug, der den Mehrschicht-Tablettenkern umgibt; und
c) magenresistenten Überzug, der den Barriereüberzug umgibt.

2. Pharmazeutische Tablettenzusammensetzung gemäß Anspruch 1, wobei der NSAID in einer freien Säureform vorliegt.

3. Pharmazeutische Tablettenzusammensetzung gemäß Anspruch 1, wobei der NSAID aus der Gruppe ausgewählt ist, welche aus Naproxen, Ibuprofen, Diclofenac, Acetylsalicylsäure und/oder Salzen und Enantiomeren davon besteht.

4. Pharmazeutische Tablettenzusammensetzung gemäß Anspruch 3, wobei der NSAID aus der Gruppe ausgewählt ist, welche aus Naproxen, Ibuprofen, Diclofenac und/oder Enantiomeren davon besteht.

5. Pharmazeutische Tablettenzusammensetzung gemäß Anspruch 3, wobei der NSAID Naproxen und/oder Naproxen-Natrium ist.

6. Pharmazeutische Tablettenzusammensetzung gemäß Anspruch 4 oder 5, wobei der NSAID Naproxen ist.

7. Pharmazeutische Tablettenzusammensetzung gemäß einem der vorhergehenden Ansprüche, wobei der PPI aus der Gruppe ausgewählt ist, welche aus Pantoprazol, Omeprazol, Lansoprazol und/oder Salzen und Enantiomeren davon besteht.

8. Pharmazeutische Tablettenzusammensetzung gemäß Anspruch 7, wobei der PPI Pantoprazol und/oder Pantoprazol-Natrium ist.

9. Pharmazeutische Tablettenzusammensetzung gemäß einem der vorhergehenden Ansprüche, wobei die NSAID-Schicht pharmazeutisch verträglichen Exzipienten umfasst, welcher aus der Gruppe ausgewählt ist, die aus Croscarmellose-Natrium, Crospovidon, Siliciumdioxid, Magnesiumstearat und/oder Povidon besteht.

10. Pharmazeutische Tablettenzusammensetzung gemäß einem der vorhergehenden Ansprüche, wobei die NSAID-Schicht Granula, umfassend NSAID und mindestens einen pharmazeutisch verträglichen Exzipienten, umfasst.

11. Pharmazeutische Tablettenzusammensetzung gemäß einem der vorhergehenden Ansprüche, wobei die PPI-Schicht zusätzlich zu multiplen Einheiten von PPI zusätzliche(n) Exzipienten, ausgewählt aus der Gruppe, welche aus Mannitol, Sorbitol, mikrokristalliner Cellulose, Crospovidon und/oder Calciumstearat besteht, umfasst.

12. Pharmazeutische Tablettenzusammensetzung gemäß einem der vorhergehenden Ansprüche, wobei multiple Einheiten von PPI einen pharmazeutisch verträglichen Exzipienten umfassen, der aus der Gruppe ausgewählt ist, welche aus Mannitol, Natriumcarbonat und/oder Crospovidon besteht.

13. Pharmazeutische Tablettenzusammensetzung gemäß einem der vorhergehenden Ansprüche, wobei der Schutzüberzug Hydroxypropylmethylcellulose, Povidon und/oder Natriumcarbonat umfasst.

14. Pharmazeutische Tablettenzusammensetzung gemäß einem der vorhergehenden Ansprüche, wobei der Barriereüberzug ein Polymer umfasst, das aus der Gruppe ausgewählt ist, welche aus Hypromellose, Povidon und/oder Polyethylenglycol besteht.

15. Pharmazeutische Tablettenzusammensetzung gemäß einem der vorhergehenden Ansprüche, wobei der magenresistente Überzug Hydroxypropylmethylcelluloseacetat und/oder Methacrylsäure-Ethylacrylat-Copolymer umfasst.

## Revendications

1. Composition de comprimé pharmaceutique comprenant :
a) un noyau de comprimé multicouche comprenant :
i) une couche de médicament anti-inflammatoire non stéroïdien (AINS) comprenant AINS et au moins un excipient pharmaceutiquement acceptable ;
ii) une couche intermédiaire optionnelle ;
iii) une couche d'inhibiteur de la pompe à protons (IPP) comprenant de multiples unités d'IPP revêtues d'un revêtement protecteur ;
b) un revêtement barrière entourant le noyau de comprimé multicouche ; et
c) un revêtement gastro-résistant entourant le revêtement barrière.

2. Composition de comprimé pharmaceutique selon la revendication 1, dans laquelle AINS est sous la forme d'acide libre.

3. Composition de comprimé pharmaceutique selon la revendication 1, dans laquelle AINS est choisi dans le groupe constitué par le naproxène, l'ibuprofène, le diclofénac, l'acide acétylsalicylique et/ou des sels et des énantiomères de ceux-ci.

4. Composition de comprimé pharmaceutique selon la revendication 3, dans laquelle AINS est choisi dans le groupe constitué par le naproxène, l'ibuprofène, le diclofénac et/ou des énantiomères de ceux-ci.

5. Composition de comprimé pharmaceutique selon la revendication 3, dans laquelle AINS est le naproxène et/ou le naproxène sodique.

6. Composition de comprimé pharmaceutique selon la revendication 4 ou 5, dans laquelle AINS est le naproxène.

7. Composition de comprimé pharmaceutique selon l'une quelconque des revendications précédentes, dans laquelle IPP est choisi dans le groupe constitué par le pantoprazole, l'oméprazole, le lansoprazole et/ou des sels et des énantiomères de ceux-ci.

8. Composition de comprimé pharmaceutique selon la revendication 7, dans laquelle IPP est le pantoprazole et/ou le pantoprazole sodique.

9. Composition de comprimé pharmaceutique selon l'une quelconque des revendications précédentes, dans laquelle la couche d'AINS comprend un excipient pharmaceutiquement acceptable choisi dans le groupe constitué par la croscarmellose sodique, la crospovidone, la silice, le stéarate de magnésium et/ou la povidone.

10. Composition de comprimé pharmaceutique selon l'une quelconque des revendications précédentes, dans laquelle la couche d'AINS comprend des granules comprenant AINS et au moins un excipient pharmaceutiquement acceptable.

11. Composition de comprimé pharmaceutique selon l'une quelconque des revendications précédentes, dans laquelle la couche d'IPP, en plus de multiples unités d'IPP, comprend un/des excipient(s) supplémentaire(s) choisi(s) dans le groupe constitué par le mannitol, le sorbitol, la cellulose microcristalline, la crospovidone et/ou le stéarate de calcium.

12. Composition de comprimé pharmaceutique selon l'une quelconque des revendications précédentes, dans laquelle de multiples unités d'IPP comprennent un excipient pharmaceutiquement acceptable qui est choisi dans le groupe constitué par le mannitol, le carbonate de sodium et/ou la crospovidone.

13. Composition de comprimé pharmaceutique selon l'une quelconque des revendications précédentes, dans laquelle le revêtement protecteur comprend de l'hydroxypropylméthylcellulose, de la povidone et/ou du carbonate de sodium.

14. Composition de comprimé pharmaceutique selon l'une quelconque des revendications précédentes, dans laquelle le revêtement barrière comprend un polymère choisi dans le groupe constitué par l'hypromellose, la povidone et/ou le polyéthylèneglycol.

15. Composition de comprimé pharmaceutique selon l'une quelconque des revendications précédentes, dans laquelle le revêtement gastro-résistant comprend de l'acétate d'hydroxypropylméthylcellulose et/ou un copolymère acide méthacrylique-acrylate d'éthyle.
